# EUROPEAN PATENT APPLICATION

(11) **EP 4 046 647 A1**
(43) Date of publication of application: **24.08.2022**
(21) Application number: 20898837.8
(22) Date of filing: 09.12.2020
(51) Int. Cl.: A61K 38/06, A61P 19/02, A61P 9/10

(54) **PHARMACEUTICAL COMPOSITION COMPRISING PEPTIDE THAT INHIBITS INTERACTION OF P53 AND FOXO4**

(30) Priority: 11.12.2019 KR 20190164447
(71) Applicant: Supadelixir Inc., Gangwon-do 24232 (KR)
(72) Inventor: HAHN, Jang-Hee, Chuncheon-si Gangwon-do 24375 (KR); KIM, Min-Seo, Chuncheon-si Gangwon-do 24377 (KR)
(74) Representative: Turner, Craig Robert
(86) International application number: PCT/KR2020/017892
(87) International publication number: WO 2021/118212

(57) **Abstract**

The present invention provides a pharmaceutical composition for preventing, ameliorating, or treating degenerative diseases caused by cellular senescence, preferably osteoarthritis or atherosclerosis, comprising a specific peptide as an active ingredient. The peptide remarkably inhibits the interaction of p53 and FOXO4 (Forkhead box protein O4), thereby inhibiting the expression of a cellular senescence regulator.

<110>    KNU-Industry Cooperation Foundation

<120>    Pharmaceutical compositions comprising a peptide inhibiting the
         interaction between p53 and FOXO4

<130>    PX0228PCT

<150>    KR 10-2019-0164447
<151>    2019-12-11

<160>    20

<170>    KoPatentIn 3.0

<210>    1
<211>    3
<212>    PRT
<213>    Artificial Sequence

<220>
<223>    Synthetic peptide


<400>    1
Leu Glu Asp
  1


<210>    2
<211>    3
<212>    PRT
<213>    Artificial Sequence

<220>
<223>    Synthetic peptide


<400>    2
Leu Ser Asp
  1


<210>    3
<211>    3
<212>    PRT
<213>    Artificial Sequence

<220>
<223>    Synthetic peptide


<400>    3
Leu Gly Asp
  1


<210>    4
<211>    3
<212>    PRT
<213>    Artificial Sequence

<220>
<223>    Synthetic peptide

<400>    4
Leu Ala Asp
1

<210>    5
<211>    3
<212>    PRT
<213>    Artificial Sequence

<220>
<223>    Synthetic peptide

<400>    5
Leu Gln Asp
1

<210>    6
<211>    3
<212>    PRT
<213>    Artificial Sequence

<220>
<223>    Synthetic peptide

<400>    6
Leu Arg Asp
1

<210>    7
<211>    3
<212>    PRT
<213>    Artificial Sequence

<220>
<223>    Synthetic peptide

<400>    7
Leu Lys Asp
1

<210>    8
<211>    3
<212>    PRT
<213>    Artificial Sequence

<220>
<223>    Synthetic peptide

<400>    8
Leu Leu Asp
1

<210>    9
<211>    3
<212>    PRT
<213>    Artificial Sequence

<220>
<223>    Synthetic peptide


<400>    9
Leu Tyr Asp
1


<210>    10
<211>    3
<212>    PRT
<213>    Artificial Sequence

<220>
<223>    Synthetic peptide


<400>    10
Leu Asp Asp
1


<210>    11
<211>    3
<212>    PRT
<213>    Artificial Sequence

<220>
<223>    Synthetic peptide


<400>    11
Leu Phe Asp
1


<210>    12
<211>    3
<212>    PRT
<213>    Artificial Sequence

<220>
<223>    Synthetic peptide


<400>    12
Leu Asn Asp
1


<210>    13
<211>    3
<212>    PRT
<213>    Artificial Sequence

<220>
<223>    Synthetic peptide

<400>    13
Leu Cys Asp
1

<210>    14
<211>    3
<212>    PRT
<213>    Artificial Sequence

<220>
<223>    Synthetic peptide

<400>    14
Leu His Asp
1

<210>    15
<211>    3
<212>    PRT
<213>    Artificial Sequence

<220>
<223>    Synthetic peptide

<400>    15
Leu Ile Asp
1

<210>    16
<211>    3
<212>    PRT
<213>    Artificial Sequence

<220>
<223>    Synthetic peptide

<400>    16
Leu Met Asp
1

<210>    17
<211>    3
<212>    PRT
<213>    Artificial Sequence

<220>
<223>    Synthetic peptide

<400>    17
Leu Pro Asp
1

<210>    18
<211>    3
<212>    PRT
<213>    Artificial Sequence

<220>
<223>    Synthetic peptide


<400>    18
Leu Thr Asp
  1


<210>    19
<211>    3
<212>    PRT
<213>    Artificial Sequence

<220>
<223>    Synthetic peptide


<400>    19
Leu Trp Asp
  1


<210>    20
<211>    3
<212>    PRT
<213>    Artificial Sequence

<220>
<223>    Synthetic peptide


<400>    20
Leu Val Asp
  1

## Description

### TECHNICAL FIELD

The present invention relates to a pharmaceutical composition comprising a peptide that inhibits the interaction between p53 and FOXO4 (Forkhead box protein O4). More specifically, the present invention relates to a pharmaceutical composition for preventing, improving, or treating degenerative diseases caused by cellular senescence, comprising a peptide having an inhibitory activity against the interaction between p53 and FOXO4 as an active ingredient.

### BACKGROUND ART

When FOXO4 receives an external stimulus such as ionizing radiation, it binds to p53 at the DNA damage site and induces cellular senescence through expression of p21, a regulator of cellular senescence. The inhibition of FOXO4-p53 binding leads to the movement of p53 to the mitochondria and the induction of cytochrome c release, thereby inducing apoptosis of senescent cells (Baar MP. et. al. (2017). targeted apoptosis of senescent cells restores tissue homeostasis in response to chemotoxicity and aging. Cell 169: 132-147, e16).

Using this mechanism, the possibilities for treating various degenerative diseases caused by cellular senescence have been studied (Childs BG et al. (2017) Senescent cells: an emerging target for diseases of aging. Nat Rev Drug Discov. 16:718-735). For example, in osteoarthritis, removal of the senescent cartilage cells accumulated on the articular cartilage surface may lead to pain relief and cartilage regeneration. In atherosclerosis, removal of the senescent foam cells, vascular smooth muscle cells, endothelial cells, etc., makes it possible to alleviate the disease by inhibiting plaque formation and lesion growth.

As such, it is expected that inhibition of the interaction between p53 and FOXO4 suppresses the expression of p21, a major regulator of cellular senescence, thereby being able to effectively prevent, improve, or treat degenerative diseases such as osteoarthritis and atherosclerosis.

### DISCLOSURE

### Technical Problem

The present inventors have found that a certain peptide remarkably increases the interaction between PKA (protein tyrosine kinase) and SHP2 (Src homology region 2 domain-containing phosphatase-2), thereby being able to inhibit cancer cell metastasis mediated by β1 integrin signaling and to be usefully applied for preventing or treating inflammatory diseases (Korean Patent Publication No. 10-2018-0099092). Surprisingly, the present inventors have found that said peptide has a preventive, ameliorating, or therapeutic activity against degenerative diseases such as osteoarthritis and atherosclerosis by remarkably inhibiting the interaction between p53 and FOXO4.

Therefore, it is an object of the present invention to provide a pharmaceutical composition for preventing, improving or treating degenerative diseases, comprising the specific peptide as an active ingredient.

### Technical Solution

In accordance with an aspect of the present invention, there is provided a pharmaceutical composition for preventing, improving or treating a degenerative disease caused by cellular senescence, comprising a peptide consisting of three amino acids of the following Formula 1 as an active ingredient:

<Formula 1> Leu-X-Asp

wherein,
X is glutamic acid (Glu), serine (Ser), glycine (Gly), alanine (Ala), glutamine (Gin), arginine (Arg), lysine (Lys), leucine (Leu), tyrosine (Tyr), aspartic acid (Asp), phenylalanine (Phe), asparagine (Asn), cysteine (Cys), histidine (His), isoleucine (Ile), methionine (Met), proline (Pro), threonine (Thr), tryptophan (Trp) , or valine (Val), and
- is a peptide bond.

In the pharmaceutical composition according to the present invention, preferably the peptide may be a peptide consisting of the amino acid sequence of SEQ ID NO: 1, 3, 4, 5, 7, 8, 9, 11, 13, 14, 15, 17, 18, 19 or 20.

In addition, in the pharmaceutical composition of the present invention, the degenerative disease caused by cellular senescence may be osteoarthritis or atherosclerosis.

### ADVANTAGEOUS EFFECTS

It has been found by the present invention that the peptide of Formula 1, i.e., the peptide consisting of three amino acids of Formula 1 remarkably inhibits the interaction between p53 and FOXO4. Accordingly, the peptide can be usefully applied to a pharmaceutical composition for preventing, improving or treating degenerative diseases such as osteoarthritis and atherosclerosis by inhibiting the expression of a cellular senescence regulator, e.g., p21.

### BRIEF DESCRIPTION OF THE DRAWINGS

FIG. 1 shows the results obtained by evaluating the effects on the interaction between p53 and FOXO4 when human keratinocytes (HaCaTs) were treated with the peptides of the present invention and active oxygen species (H₂O₂).
FIG. 2 shows the results obtained by evaluating the effects of ERK and SHP2 activation by the peptide on the interaction of p53 and FOXO4, through treating human keratinocytes (HaCaTs) with the peptide of the present invention, active oxygen species (H₂O₂), the ERK inhibitor, and the SHP2 inhibitor.
FIG. 3 shows the results obtained by evaluating, through β-Galactosidase staining, the effects on apoptosis of the senescent cells induced by active oxygen species, when human retinal pigment epithelial cells (ARPE-19) were treated with the peptide of the present invention and active oxygen species (H₂O₂).

FIG. 4 shows the results of quantitative analysis on the numbers of the remaining senescent cells confirmed through the β-Galactosidase staining in FIG. 3.

### BEST MODE

As used herein, the term "degenerative diseases" refers to diseases caused by cellular senescence, including e.g., atherosclerosis, osteoarthritis, Alzheimer's disease, Parkinson's disease, chronic obstructive pulmonary disease, idiopathic pulmonary fibrosis, retinopathy, etc., and preferably osteoarthritis and atherosclerosis.

The present invention provides a pharmaceutical composition for preventing, improving or treating a degenerative disease caused by cellular senescence, comprising a peptide consisting of three amino acids of the following Formula 1 as an active ingredient:

<Formula 1> Leu-X-Asp

wherein,
X is glutamic acid (Glu), serine (Ser), glycine (Gly), alanine (Ala), glutamine (Gln), arginine (Arg), lysine (Lys), leucine (Leu), tyrosine (Tyr), aspartic acid (Asp), phenylalanine (Phe), asparagine (Asn), cysteine (Cys), histidine (His), isoleucine (Ile), methionine (Met), proline (Pro), threonine (Thr), tryptophan (Trp) , or valine (Val), and
- is a peptide bond.

In the pharmaceutical composition according to the present invention, the peptide consisting of the amino acid sequence of SEQ ID NO: 1, 3, 4, 5, 7, 8, 9, 11, 13, 14, 15, 17, 18, 19, or 20 has been found to have a particularly excellent activity in inhibiting the interaction between p53 and FOXO4. Accordingly, in an embodiment, the peptide may be a peptide consisting of the amino acid sequence of SEQ ID NO: 1, 3, 4, 5, 7, 8, 9, 11, 13, 14, 15, 17, 18, 19, or 20.

The pharmaceutical composition of the present invention may include a pharmaceutically acceptable carrier, for example additives such as lactose or corn starch, lubricants such as magnesium stearate, currently available emulsifiers, suspending agents, buffers, isotonic agents, etc. The pharmaceutical composition of the present invention can be formulated to an oral dosage form or a parenteral dosage form, preferably to a parenteral dosage form for transdermal administration, subcutaneous administration, intramuscular administration, etc. For example, for intramuscular, subcutaneous and intravenous dosage forms, sterile solutions of the active ingredient are usually prepared and may contain a buffer capable of adjusting the pH of the solution appropriately. For intravenous administration, an isotonic agent may be included so as to render the formulation isotonic. And, the composition of the present invention may be in the form of an aqueous solution containing pharmaceutically acceptable carriers, e.g., saline having a pH level of 7.4. The solutions may be introduced into a patient's intramuscular blood-stream by local bolus injection. The pharmaceutical composition of the present invention may be administered to a patient in an amount suitable for preventing, improving, or treating a degenerative disease, e.g., at a daily dosage of about 0.01 to 10 mg/kg, which may be generally changed according to the age, body weight, and conditions of a patient.

Hereinafter, the present invention will be described more specifically by the following examples and experimental examples. However, the following examples and experimental examples are provided only for illustrations and thus the present invention is not limited to or by them.

### Example 1: Synthesis of peptides

The peptides of SEQ ID NOs: 1 to 20 (see Table 1 below) were synthesized with an automatic peptide synthesizer (PeptrEx-R48, Peptron, Daejeon, Korea) using a FMOC solid-phase method. The synthesized peptides were purified and analyzed by reverse-phase high-performance liquid chromatography (reverse-phase HPLC) (Prominence LC-20AB, Shimadzu, Japan) using a C18 analytical RP column (Shiseido capcell pak), and identified using a mass spectrometer (HP 1100 Series LC/MSD, Hewlett-Packard, Roseville, U.S.A.).

**Table 1**

| Peptide name | SEQ ID NO | Amino acid sequence |
|---|---|---|
| Pep1 | SEQ ID NO: 1 | Leu-Glu-Asp |
| Pep2 | SEQ ID NO: 2 | Leu-Ser-Asp |
| Pep3 | SEQ ID NO: 3 | Leu-Gly-Asp |
| Pep4 | SEQ ID NO: 4 | Leu-Ala-Asp |
| Pep5 | SEQ ID NO: 5 | Leu-Gln-Asp |
| Pep6 | SEQ ID NO: 6 | Leu-Arg-Asp |
| Pep7 | SEQ ID NO: 7 | Leu-Lys-Asp |
| Pep8 | SEQ ID NO: 8 | Leu-Leu-Asp |
| Pep9 | SEQ ID NO: 9 | Leu-Tyr-Asp |
| Pep10 | SEQ ID NO: 10 | Leu-Asp-Asp |
| Pep11 | SEQ ID NO: 11 | Leu-Phe-Asp |
| Pep12 | SEQ ID NO: 12 | Leu-Asn-Asp |
| Pep13 | SEQ ID NO: 13 | Leu-Cys-Asp |
| Pep14 | SEQ ID NO: 14 | Leu-His-Asp |
| Pep15 | SEQ ID NO: 15 | Leu-Ile-Asp |
| Pep16 | SEQ ID NO: 16 | Leu-Met-Asp |
| Pep17 | SEQ ID NO: 17 | Leu-Pro-Asp |
| Pep18 | SEQ ID NO: 18 | Leu-Thr-Asp |
| Pep19 | SEQ ID NO: 19 | Leu-Trp-Asp |
| Pep20 | SEQ ID NO: 20 | Leu-Val-Asp |

### Example 2: Preparation of the compositions containing peptides

The peptides of SEQ ID NOs: 1 to 20 were respectively dissolved in phosphate buffered saline (PBS) to a concentration of 1 M. The resultant protein solutions were also used in the following experimental examples.

### Experimental Example 1: Evaluation of the effects of peptides on the physical binding between p53 and FOXO4

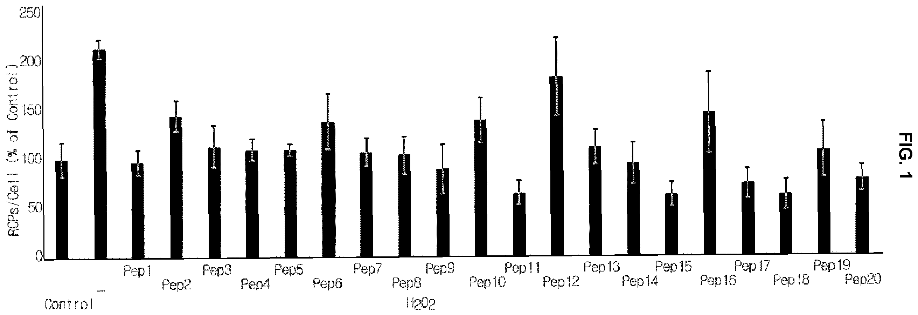

After treating cells with the peptides of the present invention, the effects thereof on the physical binding between p53 and FOXO4 were evaluated through an *in situ* PLA (proximity ligation assay) method. HaCaT cells (human keratinocytes, CLS) were added at 4.5 X 10⁴ cells per well, along with a DMEM containing 10% fetal bovine serum, to each well of a 24-well microplate and then cultured at 37°C in a 5% CO₂ incubator for 24 hours. H₂O₂ and the peptide solutions of Example 2 were added thereto so that the concentrations of H₂O₂ and each peptide in the medium were 200 µM and 20 µM respectively, followed by incubating for 1 hour under the same condition. As a control group, an untreated group (indicated by "control" in FIG. 1) and a group treated with H₂O₂ alone (indicated by "-" in FIG. 1) were used. The cells of each well were washed with PBS, fixed by treating with 2% formaldehyde for 15 minutes, and then treated with 0.1% TritonX-100 for 5 minutes to increase antibody permeability into the cells. Anti-p53 polyclonal antibody (Abcam, UK) and anti-FOXO4 polyclonal antibody (Santa Cruz, CA, USA) were added thereto. After the PLA probe was added thereto using the in situ PLA kit (Sigma-Aldrich), hybridization, ligation, amplification and mounting steps were carried out according to the manufacturer's protocol. Each physical interaction between p53 and FOXO4 was quantified by measuring the luminescence signals (PLA signals) detected in each cell with a confocal laser microscope (Olympus fluoview FW1000; Olympus, Tokyo, Japan). The results thereof are shown in FIG. 1. From the results of FIG. 1, it can be confirmed that the groups treated with the peptides of the present invention significantly inhibited the interaction between p53 and FOXO4, in comparison with the untreated control group. In particular, it can be confirmed that Pep 1, 3, 4, 5, 7, 8, 9, 11, 13, 14, 15, 17, 18, 19, or 20 (i.e., the peptides of SEQ ID NOs: 1, 3, 4, 5, 7, 8, 9, 11, 13, 14, 15, 17, 18, 19, or 20) remarkably inhibited the interaction between p53 and FOXO4, in comparison with the control group, and that Pep 1, 9, 11, 14, 15, 17, 18, and 20 (i.e., peptides of SEQ ID NOs: 1, 9, 11, 14, 15, 17, 18, and 20) remarkably inhibited the interaction between p53 and FOXO4 by two or more times, in comparison with the control group.

### Experimental Example 2: Evaluation of the effects of ERK and SHP2 on the inhibitory signal of physical binding between p53 and FOXO4 by the peptide

After treating cells with the peptide of the present invention (the peptide of SEQ ID NO: 3), H₂O₂, a ERK inhibitor (PD98059), and a SHP2 inhibitor (NSC878777), the effects thereof on the physical binding between p53 and FOXO4 were evaluated through an *in situ* PLA (proximity ligation assay) method. HaCaT cells (human keratinocytes, CLS) were added at 4.5 X 10⁴ cells per well, along with a DMEM containing 10% fetal bovine serum, to each well of a 24-well microplate and then cultured at 37°C in a 5%

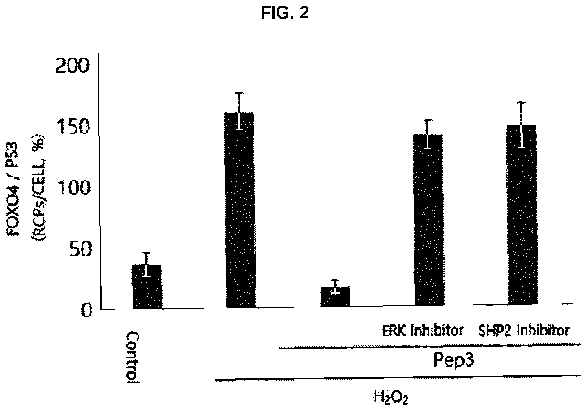

CO₂ incubator for 24 hours. The cells were treated with H₂O₂ (200 µM) and the peptide of SEQ ID NO: 3 (20 µM); or PD98059 (10 µM) or NSC878777 (10 µM) was additionally treated to the cells treated with H₂O₂ (200 µM) and the peptide of SEQ ID NO: 3 (20 µM), followed by incubating for 1 hour under the same condition. As a control group, an untreated group (indicated by "control" in FIG. 2) and a group treated with H₂O₂ alone were used. The cells of each well were washed with PBS, fixed by treating with 2% formaldehyde for 15 minutes, and then treated with 0.1% TritonX-100 for 5 minutes to increase antibody permeability into the cells. Anti-p53 polyclonal antibody (Abcam, UK) and anti-FOXO4 polyclonal antibody (Santa Cruz, CA, USA) were added thereto. After the PLA probe was added thereto using the in situ PLA kit (Sigma-Aldrich), hybridization, ligation, amplification and mounting steps were carried out according to the manufacturer's protocol. Each physical interaction between p53 and FOXO4 was quantified by measuring the luminescence signals (PLA signals) detected in each cell with a confocal laser microscope (Olympus fluoview FW1000; Olympus, Tokyo, Japan). The results thereof are shown in FIG. 2. From the results of FIG. 2, it can be confirmed that the groups treated with the peptide of the present invention significantly inhibited the interaction between p53 and FOXO4 in comparison with the untreated control group; and that the inhibitory signal by the peptide is dependent on ERK and SHP2 activation.

### Experimental Example 3: Evaluation of the activities of peptide on the apoptosis induction of senescent cells through increase in p53 activity

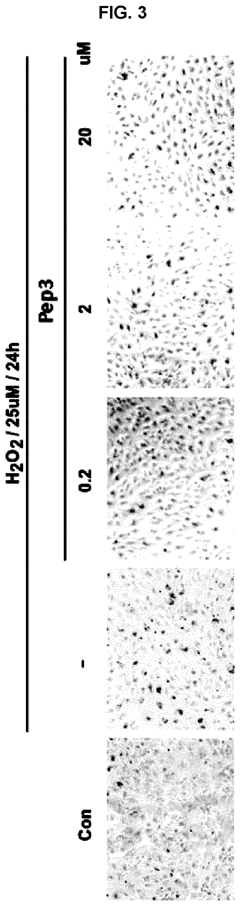

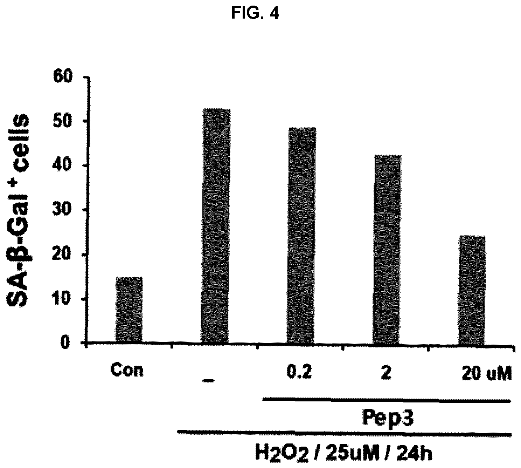

In order to evaluate the activity on the apoptosis of senescent cells by the treatment with the peptide of the present invention (the peptide of SEQ ID NO: 3) for 24 hours after inducing senescence by treating cells with H₂O₂, the X-Gal staining was carried out to measure the number of the remaining senescent cells. ARPE-19 cells (retinal pigment epithelial cells) were added at the density of 80% per well, along with a DMEM containing 10% fetal bovine serum, to each well of a 12-well microplate and then cultured at 37°C in a 5% CO₂ incubator for 24 hours. H₂O₂ was added thereto so that the concentration thereof in the medium was 25 µM, followed by incubating for 24 hours under the same condition. Thereafter, the cells in each well were washed with PBS and the peptide solution of Example 2 was added thereto so that the concentration of the peptide of SEQ ID NO: 3 was 0.2 µM, 2 µM, and 20 µM respectively, followed by incubating for 24 hours under the same condition. As a control group, an untreated group (indicated as "control" in FIGs. 3 and 4) and a group treated with H₂O₂ alone (indicated by "-" in FIGs. 3 and 4) were used. The cells in each well were washed with PBS, and X-gal staining was performed using the Senescence β-Galactosidase Staining Kit (CST) according to the manufacturer's protocol. The bluish green color development detected in the senescence-induced cells was measured with a phase-contrast microscope (Olympus, Tokyo, Japan), and each number of senescent cells was quantified. The results are shown in FIGs. 3 and 4. From the results of FIGs. 3 and 4, it can be confirmed that the groups treated with the peptide of the present invention significantly induced the apoptosis of senescent cells, in comparison with the untreated control group (i.e., the "-" group).

## Claims

1. A pharmaceutical composition for preventing, improving or treating a degenerative disease caused by cellular senescence, comprising a peptide consisting of three amino acids of the following Formula 1 as an active ingredient:
<Formula 1> Leu-X-Asp
wherein,
X is glutamic acid (Glu), serine (Ser), glycine (Gly), alanine (Ala), glutamine (Gln), arginine (Arg), lysine (Lys), leucine (Leu), tyrosine (Tyr), aspartic acid (Asp), phenylalanine (Phe), asparagine (Asn), cysteine (Cys), histidine (His), isoleucine (Ile), methionine (Met), proline (Pro), threonine (Thr), tryptophan (Trp) , or valine (Val), and
- is a peptide bond.

2. The pharmaceutical composition according to claim 1, wherein the peptide is a peptide consisting of the amino acid sequence of SEQ ID NO: 1, 3, 4, 5, 7, 8, 9, 11, 13, 14, 15, 17, 18, 19 or 20.

3. The pharmaceutical composition according to claim 1 or 2, wherein the degenerative disease caused by cellular senescence is osteoarthritis or atherosclerosis.
